# EUROPEAN PATENT APPLICATION

(11) **EP 2 324 848 A2**
(43) Date of publication of application: **25.05.2011**
(21) Application number: 10181761.7
(22) Date of filing: 21.10.2005
(51) Int. Cl.: A61K 39/00

(54) **Method for treating intraocular neovascular diseases**

(30) Priority: 21.10.2004 US 621209 P
(62) Divisional of application: 05816281.9
(71) Applicant: Genentech, Inc., South San Francisco CA 94080-4990 (US)
(72) Inventor: Shams, Naveed, Danville, CA 94506 (US)
(74) Representative: Denison, Christopher Marcus

(57) **Abstract**

A method is provided for administering a mammal suffering from, or at risk for, an intraocular neovascular disorder with regular dosing of a therapeutically effective amount of VEGF antagonist, followed by less frequent dosing of a therapeutically effective amount of VEGF antagonist.

## Description

### FIELD OF THE INVENTION

This invention relates to methods for treating an intraocular neovascular disorder with a VEGF antagonist. Methods for administering to a mammal suffering from, or at risk for, an intraocular neovascular disorder include monthly dosing of a therapeutically effective amount of VEGF antagonist, followed by less frequent dosing of a therapeutically effective amount of VEGF antagonist.

### BACKGROUND OF THE INVENTION

Angiogenesis is implicated in the pathogenesis of intraocular neovascular diseases, e.g., proliferative retinopathies, age-related macular degeneration (AMD), etc., as well as a variety of other disorders. These include solid tumors, rheumatoid arthritis, and psoriasis (Folkman et al. J. Biol. Chem. 267:10931-10934 (1992); Klagsbrun et al. Annu. Rev. Physiol. 53:217-239 (1991); and Garner A, Vascular diseases. In: Pathobiology of ocular disease. A dynamic approach. Garner A, Klintworth GK, Eds. 2nd Edition Marcel Dekker, NY, pp 1625-1710 (1994)).

The search for positive regulators of angiogenesis has yielded many candidates, including aFGF, bFGF, TGF-α, TGF-β HGF, TNF-α, angiogenin, IL-8, etc. (Folkman *et al.* and Klagsbrun *et al).* The negative regulators so far identified include thrombospondin (Good *et al. Proc. Natl.*

*Acad. Sci. USA.* 87:6624-6628 (1990)), the 16-kilodalton N-terminal fragment of prolactin (Clapp et al. Endocrinology, 133:1292-1299 (1993)), angiostatin (O'Reilly et al. Cell, 79:315-328 (1994)) and endostatin (O'Reilly et al. Cell, 88:277-285 (1996)).

Work done over the last several years has established the key role of vascular endothelial growth factor (VEGF) in the regulation of normal and abnormal angiogenesis (Ferrara et al. Endocr. Rev. 18:4-25 (1997)). The finding that the loss of even a single VEGF allele results in embryonic lethality points to an irreplaceable role played by this factor in the development and differentiation of the vascular system (Ferrara *et al.).*

Human VEGF exists as at least six isoforms (VEGF₁₂₁, VEGF₁₄₅, VEGF₁₆₅, VEGF)₈₃, VEGF)₁₈₉, and VEGF₂₀₆) that arise from alternative splicing of mRNA of a single gene (Ferrara N, Davis Smyth T. Endocr Rev 18:1-22 (1997)). VEGF₁₆₅, the most abundant isoform, is a basic, heparin binding, dimeric glycoprotein with a molecular mass of -45,000 daltons (*Id*)*.* Two VEGF receptor tyrosine kinases, VEGFR1and VEGFR2, have been identified (Shibuya et al. Oncogene 5:519-24 (1990); Matthews et al., Proc Natl Acad Sci U S A 88:9026-30 (1991); Terman et al., Oncogene 6:1677-83 (1991); Terman et al. Biochem Biophys Res Commun 187:1579-86 (1992); de Vries et al., Science 255:989-91 (1992); Millauer et al. Cell 72:835-46 (1993); and, Quinn et al. Proc Natl Acad Sci USA 90:7533-7 (1993)). VEGFRI has the highest affinity for VEGF, with a Kd of -10-20 pM (de Vries et al., Science 255:989-91 (1992)), and VEGFR2 has a somewhat lower affinity for VEGF, with a Kd of -75-125 pM (Terman et al., Oncogene 6:1677-83 (1991); Millauer et al. Cell 72:835-46 (1993); and, Quinn et al. Proc Natl Acad Sci USA 90:7533-7 (1993)).

VEGF has several biologic functions, including regulation of VEGF gene expression under hypoxic conditions (Ferrara N, Davis Smyth T. Endocr Rev 18:1-22 (1997)), mitogenic activity for micro and macrovascular endothelial cells (Ferrara N, Henzel WJ. Biochem Biophys Res Commun 161:851-8 (1989); Leung et al., Science 246:1306-9 (1989); Connolly et al. J Clin Invest 84:1470-8 (1989a); Keck et al. Science 246:1309-12 (1989); Plouet et al., EMBO J 8:3801-6 (1989); Conn et al. Proc Natl Acad Sci USA 87:2628-32 (1990); and, Pepper et al., Exp Cell Res 210:298-305 (1994)), and induction of expression of plasminogen activators and collagenase (Pepper et al., Biochem Biophys Res Commun 181:902-6 (1991)).

Furthermore, VEGF has been shown to be a key mediator of neovascularization associated with tumors and intraocular disorders (Ferrara *et al.).* The VEGF mRNA is overexpressed by the majority of human tumors examined. Berkman et al. J Clin Invest 91:153-159 (1993); Brown et al. Human Pathol 26:86-91 (1995); Brown et al. Cancer Res 53:4727-4735 (1993); Mattern et al. Brit J Cancer. 73:931-934 (1996); and Dvorak et al. Am J Pathol 146:1029-1039 (1995). Also, the concentration of VEGF in eye fluids are highly correlated to the presence of active proliferation of blood vessels in patients with diabetic and other ischemia-related retinopathies. Aiello et al., N. Engl. J. Med. 331:1480-1487 (1994). Furthermore, recent studies have demonstrated the localization of VEGF in choroidal neovascular membranes in patients affected by AMD. Lopez et al., Invest. Ophtalmo. Vis. Sci. 37:855-868 (1996); Kvanta et al., Invest Ophthalmol Vis Sci 37:1929-34 (1996).

Age related macular degeneration (AMD) is a leading cause of severe, irreversible vision loss among the elderly. Bressler, JAMA 291:1900-1 (2004). It is characterized by a broad spectrum of clinical and pathologic findings, such as pale yellow spots known as drusen, disruption of the retinal pigment epithelium (RPE), choroidal neovascularization (CNV), and disciform macular degeneration. The manifestations of the disease are classified into two forms: non exudative (dry) and exudative (wet or neovascular). Drusen are the characteristic lesions of the dry form, and neovascularization characterizes the wet form. Disciform AMD is the fibrotic stage of the neovascular lesion.

There is a dramatic increase in the prevalence of AMD with advancing age. *See, e.g.,* Leibowitz et al., Surv Ophthalmol 24(Suppl):335-610 (1980) and Klein et al., Ophthalmology 99:933-43 (1992). Although the wet form of AMD is much less common, it is responsible for 80%-90% of the severe visual loss associated with AMD (Ferris et al., Arch Ophthamol 102:1640-2 (1984)). There is an estimated 1-1.2 million prevalent cases of wet AMD. The cause of AMD is unknown; however, it is clear that the risk of developing AMD increases with advancing age. Other known risk factors include family history and cigarette smoking. Postulated risk factors also include oxidative stress, diabetes, alcohol intake, and sunlight exposure. D'Amico, N Engl J Med 331:95-106 (1994) and Christen et al., JAMA 276:1147-51 (1996).

Dry AMD is characterized by changes in the RPE and Bruch's membrane. It is thought that the RPE, compromised by age and other risk factors, deposits lipofuscin and cellular debris on Bruch's membrane. These changes may be seen ophthalmoscopically as drusen, which are scattered throughout the macula and posterior retinal pole. There are also variable degrees of atrophy and pigmentation of the RPE. Dry AMD may be asymptomatic or accompanied by variable and usually minimal visual loss and is considered to be a prelude to development of wet AMD.

Wet AMD is typically characterized by CNV of the macular region. The choroidal capillaries proliferate and penetrate Bruch's membrane to reach the RPE and may extend into the subretinal space. The increased permeability of the newly formed capillaries leads to accumulation of serous fluid or blood under the RPE and/or the neurosensory retina or within the neurosensory retina. When the fovea becomes swollen or detached, decreases in vision occur. Fibrous metaplasia and organization may ensue, resulting in an elevated subretinal mass called a disciform scar that constitutes end-stage AMD and is associated with permanent vision loss (D'Amico DJ. N Engl J Med 331:95-106 (1994)).

The neovascularization in AMD can be classified into different patterns based on fluorescein angiography of subfoveal chorodial neovascular lesions. TAP and VIP Study Groups, Arch Ophthalmol 121:1253-68 (2003). The major angiographic patterns are termed classic and occult and are associated with different degrees of aggressiveness, vision losses, and response to different treatment options.

The diffusible nature of VEGF and its specificity of action for endothelial cells support a key role in the process of abnormal blood vessel growth and vascular leakage. Increased expression of VEGF in retinal photoreceptors or RPE of transgenic mice stimulates neovascularization within the retina, and VEGF antagonists partially inhibit retinal neovascularization in animal models (Okamoto et al. Am J Pathol 151:281-91 (1997); Schwesinger et al., AM J Pathol. Mar;158(3):1161-72 (2001)). Anti-VEGF neutralizing antibodies inhibit intraocular angiogenesis in models of ischemic retinal disorders (Adamis et al. Arch. Ophthalmol. 114:66-71 (1996)), and also suppress the growth of a variety of human tumor cell lines in nude mice (Kim et al. Nature 362:841-844 (1993); Warren et al. J. Clin. Invest. 95:1789-1797 (1995); Borgström et al. Cancer Res. 56:4032-4039 (1996); and Melnyk et al. Cancer Res. 56:921-924 (1996)). Therefore, anti-VEGF monoclonal antibodies or other VEGF antagonists are promising candidates for use in treatments of intraocular neovascular disorders, and new methods of administering therapeutic compounds, which increases the effectiveness of the therapeutic compound, are needed.

### SUMMARY OF THE INVENTION

One object of the present invention is to provide an improved method of administering a therapeutic compound. This and other objects will become apparent from the following description.

Methods for treating intraocular neovascular disease are provided. For example, methods include administering to a mammal a number of first individual doses of a VEGF antagonist, followed by administering to the mammal a number of second individual doses of the antagonist, wherein the second individual doses are administered less frequently than the first individual doses.

In one embodiment of the invention, a method for treating wet form age-related macular degeneration is provided, which comprises administering to a mammal a number of first individual doses of an VEGF antagonist, followed by administering to the mammal a number of second individual doses of the antagonist, wherein the second individual doses are administered less frequently than the first individual doses.

In one embodiment, the mammal is in need of treatment. Typically, the mammal is a human.

In one embodiment, the administration of the VEGF antagonist is ocular. In one aspect, the administration is intraocular. In another aspect, the administration is intravitreal.

A VEGF antagonist is administered in the methods of the invention. In one aspect, the VEGF antagonist is an anti-VEGF antibody, e.g., a full length anti-VEGF antibody or an antibody fragment. In one embodiment, the anti-VEGF antibody is a Fab antibody fragment. In one embodiment, the antibody fragment is Y0317.

In one embodiment of the invention, the first individual doses are administered at one month intervals (e.g., about 3 individual doses). Typically, there is more than one first individual dose. In another embodiment, the second individual doses are administered at three month intervals (e.g., about 6 individual doses). In one aspect of the invention, the second individual doses are administered beginning three months after the number of first individual doses. In one embodiment, a number of second individual doses are administered to the mammal during a period of at least 22 months following the number of first individual doses.

In one embodiment of the invention, the number of first individual doses and the number of second individual doses are administered over a time period of about 2 years. In one aspect, the first individual dose is administered at month 0, 1 and 2. In another aspect, the second individual dose is administered at month 5, 8, 11, 14, 17, 20 and 23. For example, the first individual dose is administered at month 0, 1, and 2 and the second individual dose is administered at month 5, 8, 11, 14, 17, 20 and 23. In one embodiment, the VEGF antagonist is administered over less than 2 years, or optionally, administered over greater than 2 years. Other aspects of the invention will become apparent from the following description of the embodiments which are not intended to be limiting of the invention.

### BRIEF DESCRIPTION OF THE FIGURES

**Figure 1** schematically illustrates the study in Example 1.
**Figure 2** schematically illustrates a dosing regimen for treating, e.g., age-related macular degeneration (AMD) with a VEGF antagonist.

### DETAILED DESCRIPTION

### Definitions

Before describing the present invention in detail, it is to be understood that this invention is not limited to particular compositions or biological systems, which can, of course, vary. It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a molecule" optionally includes a combination of two or more such molecules, and the like.

The term "human VEGF" as used herein refers to the 165-amino acid human vascular endothelial cell growth factor, and related 121-, 189-, and 206-, (and other isoforms) amino acid vascular endothelial cell growth factors, as described by Leung et al., Science 246:1306 (1989), and Houck et al., Mol. Endocrin. 5:1806 (1991) together with the naturally occurring allelic and processed forms of those growth factors.

A "VEGF antagonist" refers to a molecule capable of neutralizing, blocking, inhibiting, abrogating, reducing or interfering with VEGF activities including its binding to one or more VEGF receptors. VEGF antagonists include anti-VEGF antibodies and antigen-binding fragments thereof, receptor molecules and derivatives which bind specifically to VEGF thereby sequestering its binding to one or more receptors, anti-VEGF receptor antibodies and VEGF receptor antagonists such as small molecule inhibitors of the VEGFR tyrosine kinases, and fusions proteins, e.g., VEGF-Trap (Regeneron), VEGF₁₂₁-gelonin (Peregrine). VEGF antagonists also include antagonist variants of VEGF, antisense molecules directed to VEGF, RNA aptamers specific to VEGF, and ribozymes against VEGF or VEGF receptors. Antagonists of VEGF act by interfering with the binding of VEGF to a cellular receptor, by incapacitating or killing cells which have been activated by VEGF, or by interfering with vascular endothelial cell activation after VEGF binding to a cellular receptor. All such points of intervention by a VEGF antagonist shall be considered equivalent for purposes of this invention. Preferred VEGF antagonists are anti-VEGF antagonistic antibodies capable of inhibiting one or more of the biological activities of VEGF, for example, its mitogenic, angiogenic or vascular permeability activity. Anti-VEGF antagonistic antibodies include, but not limited to, antibodies A4.6.1, rhuMab VEGF (bevacizumab), Y0317 (ranibizumab), G6, B20, 2C3, and others as described in, for example, WO98/45331, US2003/0190317, U.S. Patents 6,582,959 and 6,703,020; WO98/45332; WO 96/30046; WO94/10202; WO2005/044853; EP 0666868B 1; and Popkov et al., Journal of Immunological Methods 288:149-164 (2004). More preferably, the anti-VEGF antagonistic antibody of the invention is ranibizumab, which is a humanized, affinity matured anti-human VEGF antibody Fab fragment having the light and heavy chain variable domain sequences of Y0317 as described in WO98/45331 and Chen et al J Mol Biol 293:865-881 (1999).

The antibody is appropriately from any source, including chicken and mammalian such as rodent, goat, primate, and human. Typically, the antibody is from the same species as the species to be treated, and more preferably the antibody is human or humanized and the host is human. While the antibody can be a polyclonal or monoclonal antibody, typically it is a monoclonal antibody, which can be prepared by conventional technology. The antibody is an IgG-1, -2, -3, or -4, IgE, IgA, IgM, IgD, or an intraclass chimera in which Fv or a CDR from one class is substituted into another class. The antibody may have an Fc domain capable of an effector function or may not be capable of binding complement or participating in ADCC.

The term "VEGF receptor" or "VEGFr" as used herein refers to a cellular receptor for VEGF, ordinarily a cell-surface receptor found on vascular endothelial cells, as well as variants thereof which retain the ability to bind hVEGF. One example of a VEGF receptor is the *fms-*like tyrosine kinase *(flt),* a transmembrane receptor in the tyrosine kinase family. DeVries et al., Science 255:989 (1992); Shibuya et al., Oncogene 5:519 (1990). The *flt* receptor comprises an extracellular domain, a transmembrane domain, and an intracellular domain with tyrosine kinase activity. The extracellular domain is involved in the binding of VEGF, whereas the intracellular domain is involved in signal transduction. Another example of a VEGF receptor is the *flk-1* receptor (also referred to as KDR). Matthews et al., Proc. Nat. Acad. Sci. 88:9026 (1991); Terman et al., Oncogene 6:1677 (1991); Terman et al., Biochem. Biophys. Res. Commun. 187:1579 (1992). Binding of VEGF to the *flt* receptor results in the formation of at least two high molecular weight complexes, having apparent molecular weight of 205,000 and 300,000 Daltons. The 300,000 Dalton complex is believed to be a dimer comprising two receptor molecules bound to a single molecule of VEGF.

The term "epitope A4.6.1" when used herein, unless indicated otherwise, refers to the region of human VEGF to which the A4.6.1 antibody disclosed in Kim et al., Growth Factors 7:53 (1992) and Kim et al. Nature 362:841 (1993), binds.

"Treatment" refers to both therapeutic treatment and prophylactic or preventative measures. Those in need of treatment include those already with the disorder as well as those in which the disorder is to be prevented.

"Mammal" for purposes of treatment refers to any animal classified as a mammal, including humans, domestic and farm animals, and zoo, sports, or pet animals, such as dogs, horses, cats, cows, *etc.* Typically, the mammal is human.

The term "antibody" is used in the broadest sense and includes monoclonal antibodies (including full length or intact monoclonal antibodies), polyclonal antibodies, multivalent antibodies, multispecific antibodies (e.g., bispecific antibodies), and antibody fragments (see below) so long as they exhibit the desired biological activity.

Unless indicated otherwise, the expression "multivalent antibody" is used throughout this specification to denote an antibody comprising three or more antigen binding sites. The multivalent antibody is typically engineered to have the three or more antigen binding sites and is generally not a native sequence IgM or IgA antibody.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges.

Each heavy chain has at one end a variable domain (V_{H} ) followed by a number of constant domains. Each light chain has a variable domain at one end (V_{L}) and a constant domain at its other end; the constant domain of the light chain is aligned with the first constant domain of the heavy chain, and the light- chain variable domain is aligned with the variable domain of the heavy chain. Particular amino acid residues are believed to form an interface between the light- and heavy-chain variable domains.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework region (FR). The variable domains of native heavy and light chains each comprise four FRs (FR1, FR2, FR3 and FR4, respectively), largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991), pages 647-669). The constant domains are not involved directly in binding an antibody to an antigen, but exhibit various effector functions, such as participation of the antibody in antibody-dependent cellular toxicity (ADCC).

The term "hypervariable region" when used herein refers to the amino acid residues of an antibody which are responsible for antigen-binding. The hypervariable region comprises amino acid residues from a "complementarity determining region" or "CDR" (*i.e.* residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (i.e. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J. Mol. Biol. 196:901-917 (1987)). "Framework" or "FR" residues are those variable domain residues other than the hypervariable region residues as herein defined.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')₂ fragment that has two antigen-combining sites and is still capable of cross-linking antigen.

"Fv" is the minimum antibody fragment which contains a complete antigen-recognition and - binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxyl terminus of the heavy chain CH1 domain including one or more cysteine(s) from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgGI, IgG2, IgG3, IgG4, IgA, and IgA2. The heavy-chain constant domains that correspond to the different classes of immunoglobulins are called α, δ, ε, γ, and µ, respectively. The subunit structures and three-dimensional configurations of different classes of immunoglobulins are well known.

"Antibody fragments" comprise only a portion of an intact antibody, generally including an antigen binding site of the intact antibody and thus retaining the ability to bind antigen. Examples of antibody fragments encompassed by the present definition include: (i) the Fab fragment, having VL, CL, VH and CH1 domains; (ii) the Fab' fragment, which is a Fab fragment having one or more cysteine residues at the C-terminus of the CH1 domain; (iii) the Fd fragment having VH and CH1 domains; (iv) the Fd' fragment having VH and CH1 domains and one or more cysteine residues at the C-terminus of the CH1 domain; (v) the Fv fragment having the VL and VH domains of a single arm of an antibody; (vi) the dAb fragment (Ward et al., Nature 341, 544-546 (1989)) which consists of a VH domain; (vii) isolated CDR regions; (viii) F(ab')2 fragments, a bivalent fragment including two Fab' fragments linked by a disulphide bridge at the hinge region; (ix) single chain antibody molecules (e.g. single chain Fv; scFv) (Bird et al., Science 242:423-426 (1988); and Huston et al., PNAS (USA) 85:5879-5883 (1988)); (x) "diabodies" with two antigen binding sites, comprising a heavy chain variable domain (VH) connected to a light chain variable domain (VL) in the same polypeptide chain (see, e.g., EP 404,097; WO 93/11161; and Hollinger et al., Proc. Natl. Acad. Sci. USA, 90:6444-6448 (1993)); (xi) "linear antibodies" comprising a pair of tandem Fd segments (VH-CH1-VH-CH1) which, together with complementary light chain polypeptides, form a pair of antigen binding regions (Zapata et al. Protein Eng. 8(10):1057 1062 (1995); and US Patent No. 5,641,870).

The term. "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature 256:495 (1975), or may be made by recombinant DNA methods (see, *e.g.,* U.S. Patent No. 4,816,567). The "monoclonal antibodies" may also be isolated from phage antibody libraries using the techniques described in Clackson et al., Nature 352:624-628 (1991) and Marks et al., J. Mol. Biol. 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (e.g., murine) antibodies are chimeric antibodies which contain minimal sequence derived from non-human immunoglobulin. For the most part, humanized antibodies are human immunoglobulins (recipient antibody) in which hypervariable region residues of the recipient are replaced by hypervariable region residues from a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues which are not found in the recipient antibody or in the donor antibody. These modifications are made to further refine antibody performance. In general, the humanized antibody will comprise substantially all of at least one, and typically two, variable domains, in which all or substantially all of the hypervariable regions correspond to those of a non-human immunoglobulin and all or substantially all of the FRs are those of a human immunoglobulin sequence. The humanized antibody optionally also will comprise at least a portion of an immunoglobulin constant region (Fc), typically that of a human immunoglobulin. For further details, see Jones et al., Nature 321:522-525 (1986); Reichmann et al., Nature 332:323-329 (1988); and Presta, Curr. Op. Struct. Biol. 2:593-596 (1992).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues. Human antibodies can be produced using various techniques known in the art. In one embodiment, the human antibody is selected from a phage library, where that phage library expresses human antibodies (Vaughan et al. Nature Biotechnology 14:309-314 (1996): Sheets et al. PNAS (USA) 95:6157-6162 (1998)); Hoogenboom and Winter, J. Mol. BioL, 227:381 (1991); Marks et al., J. Mol. Biol., 222:581 (1991)). Human antibodies can also be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10: 779-783 (1992); Lonberg et al., Nature 368: 856-859 (1994); Morrison, Nature 368:812-13 (1994); Fishwild et al., Nature Biotechnology 14: 845-51 (1996); Neuberger, Nature Biotechnology 14: 826 (1996); Lonberg and Huszar, Intern. Rev. Immunol. 13:65-93 (1995). Alternatively, the human antibody may be prepared via immortalization of human B lymphocytes producing an antibody directed against a target antigen (such B lymphocytes may be recovered from an individual or may have been immunized *in vitro). See, e.g.,* Cole et al., Monoclonal Antibodies and Cancer Therapy, Alan R. Liss, p. 77 (1985); Boerner et al., J. Immunol., 147 (1):86-95 (1991); and US Pat No. 5,750,373.

The term "Fc region" is used to define the C-terminal region of an immunoglobulin heavy chain which may be generated by papain digestion of an intact antibody. The Fc region may be a native sequence Fc region or a variant Fc region. Although the boundaries of the Fc region of an immunoglobulin heavy chain might vary, the human IgG heavy chain Fc region is usually defined to stretch from an amino acid residue at about position Cys226, or from about position Pro230, to the carboxyl-terminus of the Fc region. The Fc region of an immunoglobulin generally comprises two constant domains, a CH2 domain and a CH3 domain, and optionally comprises a CH4 domain.

By "Fc region chain" herein is meant one of the two polypeptide chains of an Fc region.

The "CH2 domain" of a human IgG Fc region (also referred to as "Cg2" domain) usually extends from an amino acid residue at about position 231 to an amino acid residue at about position 340. The CH2 domain is unique in that it is not closely paired with another domain. Rather, two N-linked branched carbohydrate chains are interposed between the two CH2 domains of an intact native IgG molecule. It has been speculated that the carbohydrate may provide a substitute for the domain-domain pairing and help stabilize the CH2 domain. Burton, Molec. Immunol.22:161-206 (1985). The CH2 domain herein may be a native sequence CH2 domain or variant CH2 domain.

The "CH3 domain" comprises the stretch of residues C-terminal to a CH2 domain in an Fc region (i.e. from an amino acid residue at about position 341 to an amino acid residue at about position 447 of an IgG). The CH3 region herein may be a native sequence CH3 domain or a variant CH3 domain (e.g. a CH3 domain with an introduced "protroberance" in one chain thereof and a corresponding introduced "cavity" in the other chain thereof; see US Patent No. 5,821,333, expressly incorporated herein by reference). Such variant CH3 domains may be used to make multispecific (e.g. bispecific) antibodies as herein described.

"Hinge region" is generally defined as stretching from about Glu216, or about Cys226, to about Pro230 of human IgG1 (Burton, Molec. Immunol.22:161-206 (1985)). Hinge regions of other IgG isotypes may be aligned with the IgG1 sequence by placing the first and last cysteine residues forming inter-heavy chain S-S bonds in the same positions. The hinge region herein may be a native sequence hinge region or a variant hinge region. The two polypeptide chains of a variant hinge region generally retain at least one cysteine residue per polypeptide chain, so that the two polypeptide chains of the variant hinge region can form a disulfide bond between the two chains. The preferred hinge region herein is a native sequence human hinge region, e.g. a native sequence human IgG1 hinge region.

A "functional Fc region" possesses at least one "effector function" of a native sequence Fc region. Exemplary "effector functions" include C1q binding; complement dependent cytotoxicity (CDC); Fc receptor binding; antibody-dependent cell-mediated cytotoxicity (ADCC); phagocytosis; down regulation of cell surface receptors (e.g. B cell receptor; BCR), etc. Such effector functions generally require the Fc region to be combined with a binding domain (e.g. an antibody variable domain) and can be assessed using various assays known in the art for evaluating such antibody effector functions.

A "native sequence Fc region" comprises an amino acid sequence identical to the amino acid sequence of an Fc region found in nature.

A "variant Fc region" comprises an amino acid sequence which differs from that of a native sequence Fc region by virtue of at least one amino acid modification. Preferably, the variant Fc region has at least one amino acid substitution compared to a native sequence Fc region or to the Fc region of a parent polypeptide, e.g. from about one to about ten amino acid substitutions, and preferably from about one to about five amino acid substitutions in a native sequence Fc region or in the Fc region of the parent polypeptide. The variant Fc region herein will typically possess, e.g., at least about 80% sequence identity with a native sequence Fc region and/or with an Fc region of a parent polypeptide, or at least about 90% sequence identity therewith, or at least about 95% sequence or more identity therewith.

"Antibody-dependent cell-mediated cytotoxicity" and "ADCC" refer to a cell-mediated reaction in which nonspecific cytotoxic cells that express Fc receptors (FcRs) (e.g. Natural Killer (NK) cells, neutrophils, and macrophages) recognize bound antibody on a target cell and subsequently cause lysis of the target cell. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol 9:457-92 (1991). To assess ADCC activity of a molecule of interest, an in vitro ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in vivo, e.g., in a animal model such as that disclosed in Clynes et al. PNAS (USA) 95:652-656 (1998).

"Human effector cells" are leukocytes which express one or more FcRs and perform effector functions. Typically, the cells express at least FcγRIII and perform ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being generally preferred. The effector cells may be isolated from a native source thereof, e.g. from blood or PBMCs as described herein.

The terms "Fc receptor" and "FcR" are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain (reviewed in Daëron, Annu. Rev. Immunol. 15:203-234 (1997)). FcRs are reviewed in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein. The term also includes the neonatal receptor, FcRn, which is responsible for the transfer of maternal IgGs to the fetus (Guyer et al., J. Immunol. 117:587 (1976); and Kim et al., J. Immunol. 24:249 (1994)).

"Complement dependent cytotoxicity" and "CDC" refer to the lysing of a target in the presence of complement. The complement activation pathway is initiated by the binding of the first component of the complement system (C1q) to a molecule (e.g. an antibody) complexed with a cognate antigen. To assess complement activation, a CDC assay, e.g. as described in Gazzano-Santoro et al., J. Immunol. Methods 202:163 (1996), may be performed.

An "affinity matured" antibody is one with one or more alterations in one or more CDRs thereof which result an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. BiolTechnology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

A "flexible linker" herein refers to a peptide comprising two or more amino acid residues joined by peptide bond(s), and provides more rotational freedom for two polypeptides (such as two Fd regions) linked thereby. Such rotational freedom allows two or more antigen binding sites joined by the flexible linker to each access target antigen(s) more efficiently. Examples of suitable flexible linker peptide sequences include gly-ser, gly-ser-gly-ser, ala-ser, and gly-gly-gly-ser.

"Single-chain Fv" or "sFv" antibody fragments comprise the V_{H} and V_{L} domains of antibody, wherein these domains are present in a single polypeptide chain. Generally, the Fv polypeptide further comprises a polypeptide linker between the V_{H} and V_{L} domains which enables the sFv to form the desired structure for antigen binding. For a review of sFv see Pluckthun in The Pharmacology of Monoclonal Antibodies, vol. 113, Rosenburg and Moore eds. Springer-Verlag, New York, pp. 269-315 (1994).

The term "diabodies" refers to small antibody fragments with two antigen-binding sites, which fragments comprise a heavy chain variable domain (V_{H}) connected to a light chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93111161; and Hollinger et al., Proc. Natl. Acad. Sci. USA 90:6444-6448 (1993).

The expression "linear antibodies" when used throughout this application refers to the antibodies described in Zapata et al. Protein Eng. 8(10):1057-1062 (1995). Briefly, these antibodies comprise a pair of tandem Fd segments (V_{H}-C_{H}1-V_{H}-C_{H}1) which form a pair of antigen binding regions. Linear antibodies can be bispecific or monospecific.

A "variant" anti-VEGF antibody, refers herein to a molecule which differs in amino acid sequence from a "parent" anti-VEGF antibody amino acid sequence by virtue of addition, deletion and/or substitution of one or more amino acid residue(s) in the parent antibody sequence. In the preferred embodiment, the variant comprises one or more amino acid substitution(s) in one or more hypervariable region(s) of the parent antibody. For example, the variant may comprise at least one, e.g. from about one to about ten, and preferably from about two to about five, substitutions in one or more hypervariable regions of the parent antibody. Ordinarily, the variant will have an amino acid sequence having at least 75% amino acid sequence identity with the parent antibody heavy or light chain variable domain sequences, more preferably at least 80%, more preferably at least 85%, more preferably at least 90%, and most preferably at least 95%. Identity or homology with respect to this sequence is defined herein as the percentage of amino acid residues in the candidate sequence that are identical with the parent antibody residues, after aligning the sequences and introducing gaps, if necessary, to achieve the maximum percent sequence identity. None of N-terminal, C-terminal, or internal extensions, deletions, or insertions into the antibody sequence shall be construed as affecting sequence identity or homology. The variant retains the ability to bind human VEGF and preferably has properties which are superior to those of the parent antibody. For example, the variant may have a stronger binding affinity, enhanced ability to inhibit VEGF-induced proliferation of endothelial cells and/or increased ability to inhibit VEGF-induced angiogenesis *in vivo.* To analyze such properties, one should compare a Fab form of the variant to a Fab form of the parent antibody or a full length form of the variant to a full length form of the parent antibody, for example, since it has been found that the format of the anti-VEGF antibody impacts its activity in the biological activity assays disclosed, e.g., in WO98/45331 and US2003/0190317. In one embodiment, the variant antibody is one which displays at least about 10 fold, preferably at least about 20 fold, and most preferably at least about 50 fold, enhancement in biological activity when compared to the parent antibody.

The "parent" antibody herein is one which is encoded by an amino acid sequence used for the preparation of the variant. Preferably, the parent antibody has a human framework region and, if present, has human antibody constant region(s). For example, the parent antibody may be a humanized or human antibody.

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

The term "epitope tagged" when used herein refers to the anti-VEGF antibody fused to an "epitope tag." The epitope tag polypeptide has enough residues to provide an epitope against which an antibody thereagainst can be made, yet is short enough such that it does not interfere with activity of the VEGF antibody. The epitope tag preferably is sufficiently unique so that the antibody thereagainst does not substantially cross-react with other epitopes. Suitable tag polypeptides generally have at least 6 amino acid residues and usually between about 8-50 amino acid residues (preferably between about 9-30 residues). Examples include the flu HA tag polypeptide and its antibody 12CA5 (Field et al. Mol. Cell. Biol. 8:2159-2165 (1988)); the c-myc tag and the 8F9, 3C7, 6E10, G4, B7 and 9E10 antibodies thereto (Evan et al., Mol. Cell. Biol. 5(12):3610-3616 (1985)); and the Herpes Simplex virus glycoprotein D (gD) tag and its antibody (Paborsky et al., Protein Engineering 3(6):547-553 (1990)). In certain embodiments, the epitope tag is a "salvage receptor binding epitope". As used herein, the term "salvage receptor binding epitope" refers to an epitope of the Fc region of an IgG molecule (e.g., IgG₁, IgG₂, IgG₃, or IgG₄) that is responsible for increasing the *in vivo* serum half-life of the IgG molecule.

An "angiogenic factor or agent" is a growth factor which stimulates the development of blood vessels, e.g., promotes angiogenesis, endothelial cell growth, stability of blood vessels, and/or vasculogenesis, etc. For example, angiogenic factors, include, but are not limited to, e.g., VEGF and members of the VEGF family, P1GF, PDGF family, fibroblast growth factor family (FGFs), TIE ligands (Angiopoietins), ephrins, ANGPTL3, ANGPTL4, etc. It would also include factors that accelerate wound healing, such as growth hormone, insulin-like growth factor-I (IGF-I), VIGF, epidermal growth factor (EGF), CTGF and members of its family, and TGF-α and TGF-β. *See, e.g.,* Klagsbrun and D'Amore, Annu. Rev. Physiol., 53:217-39 (1991); Streit and Detmar, Oncogene, 22:3172-3179 (2003); Ferrara & Alitalo, Nature Medicine 5(12):1359-1364 (1999); Tonini et al., Oncogene, 22:6549-6556 (2003) (e.g., Table 1 listing angiogenic factors); and, Sato Int. J. Clin. Oncol., 8:200-206 (2003).

An "anti-angiogenesis agent" or "angiogenesis inhibitor" refers to a small molecular weight substance, a polynucleotide, a polypeptide, an isolated protein, a recombinant protein, an antibody, or conjugates or fusion proteins thereof, that inhibits angiogenesis, vasculogenesis, or undesirable vascular permeability, either directly or indirectly. For example, an anti-angiogenesis agent is an antibody or other antagonist to an angiogenic agent as defined above, e.g., antibodies to VEGF, antibodies to VEGF receptors, small molecules that block VEGF receptor signaling (e.g., PTK787/ZK2284, SU6668). Anti-angiogensis agents also include native angiogenesis inhibitors, e.g., angiostatin, endostatin, etc. *See, e.g.,* Klagsbrun and D'Amore, Annu. Rev. Physiol., 53:217-39 (1991); Streit and Detmar, Oncogene, 22:3172-3179 (2003) (e.g., Table 3 listing anti-angiogenic therapy in malignant melanoma); Ferrara & Alitalo, Nature Medicine 5(12):1359-1364 (1999); Tonini et al., Oncogene, 22:6549-6556 (2003) (e.g., Table 2 listing antiangiogenic factors); and, Sato Int. J. Clin. Oncol., 8:200-206 (2003) (e.g., Table 1 lists Anti-angiogenic agents used in clinical trials).

The term "effective amount" or "therapeutically effective amount" refers to an amount of a drug effective to treat a disease or disorder in a mammal. In the case of age-related macular degeneration (AMD), the effective amount of the drug can reduce or prevent vision loss. For AMD therapy, efficacy in vivo can, for example, be measured by one or more of the following: assessing the mean change in the best corrected visual acuity (BCVA) from baseline to a desired time, assessing the proportion of subjects who lose fewer than 15 letters in visual acuity at a desired time compared with baseline, assessing the proportion of subjects who gain greater than or equal to 15 letters in visual acuity at a desired time compared with baseline, assessing the proportion of subjects with a visual-acuity Snellen equivalent of 20/2000 or worse at desired time, assessing the NEI Visual Functioning Questionnaire, assessing the size of CNV and amount of leakage of CNV at a desired time , as assessed by fluorescein angiography, etc.

A therapeutic dose is a dose which exhibits a therapeutic effect on the patient and a subtherapeutic dose is a dose which does not exhibit a therapeutic effect on the patient treated. An "intraocular neovascular disease" is a disease characterized by ocular neovascularization. Examples of intraocular neovascular diseases include, but are not limited to, e.g., proliferative retinopathies, choroidal neovascularization (CNV), age-related macular degeneration (AMD), diabetic and other ischemia-related retinopathies, diabetic macular edema, pathological myopia, von Hippel-Lindau disease, histoplasmosis of the eye, Central Retinal Vein Occlusion (CRVO), corneal neovascularization, retinal neovascularization, etc.

The word "label" when used herein refers to a detectable compound or composition which is conjugated directly or indirectly to the antibody. The label may itself be detectable by itself (e.g., radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g. controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g. an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as the anti-VEGF antibodies) to a mammal. The components of the liposome are commonly arranged in a bilayer formation, similar to the lipid arrangement of biological membranes.

### MODES OF THE INVENTION

It has been discovered that the treatment effects of a VEGF antagonist, e.g., Ranibizumab, are maintained for an extended period of time, such as more than one month. Treatment with the VEGF antagonist was also found to be well tolerated for up to 2 years. The present invention describes a treatment schedule comprising an initial interval of administration of a therapeutic compound, followed by a subsequent, less frequent interval of administration of the therapeutic compound. The methods of the present invention allow one to decrease subsequent doses of the therapeutic compound, while at the same time maintaining the therapeutic efficacy.

The therapeutic compound which is administered using the treatment schedule of the present invention is a VEGF antagonist, preferably an anti-VEGF antibody (e.g., Ranibizumab). VEGF is a secreted homodimeric protein that is a potent vascular endothelial cells mitogen (Ferrara N, Davis Smyth T. Endocr Rev 18:1-22 (1997). VEGF stimulates vascular endothelial cell growth, functions as a survival factor for newly formed vessels, and induces vascular permeability. VEGF expression is upregulated by hypoxia as well as by a number of other stimuli.

In methods of the invention, therapeutic effects of a VEGF antagonist are provided by administering to a mammal a number of first individual doses of an VEGF antagonist; followed by, administering to the mammal a number of second individual doses of the antagonist, where the second individual doses are administered less frequently than the first individual doses.

The term "therapeutic" in this context means that the compounds binds to the ligand, VEGF, and produce a change in the symptoms or conditions associated with the disease or condition which is being treated. It is sufficient that a therapeutic dose produce an incremental change in the symptoms or conditions associated with the disease; a cure or complete remission of symptoms is not required. One having ordinary skill in this art can easily determine whether a dose is therapeutic by establishing criteria for measuring changes in symptoms or conditions of the disease being treated and then monitoring changes in these criteria according to known methods. External physical conditions, histologic examination of affected tissues in patients or the presence or absence of specific cells or compounds, associated with a disease may provide objective criteria for evaluating therapeutic effect. In one example, methods of the invention may be used to treat AMD where therapeutic effect is assessed by changes in preventing vision loss. Other indicators of therapeutic effect will be readily apparent to one having ordinary skill in the art and may be used to establish efficacy of the dose. See also section entitled herein, "Efficacy of the Treatment."

The doses may be administered according to any time schedule which is appropriate for treatment of the disease or condition. For example, the dosages may be administered on a daily, weekly, biweekly or monthly basis in order to achieve the desired therapeutic effect and reduction in adverse effects. The dosages can be administered before, during or after the development of the disorder. The specific time schedule can be readily determined by a physician having ordinary skill in administering the therapeutic compound by routine adjustments of the dosing schedule within the method of the present invention. The time of administration of the number of first individual and second individual doses as well as subsequent dosages is adjusted to minimize adverse effects while maintaining a maximum therapeutic effect. The occurrence of adverse effects can be monitored by routine patient interviews and adjusted to minimize the occurrence of side effects by adjusting the time of the dosing. Any dosing time is to be considered to be within the scope of the present invention so long as the number of first individual doses of the VEGF antagonist is administered followed by a number of second individual doses, which are less frequently administered. For example, doses may be administered on a monthly schedule followed by subsequent quarterly or more dose schedule. Maintenance doses are also contemplated by the invention.

In a further embodiment, the first individual dose may be repeated one or more times before the second individual dose is administered. The first dose may be administered, for example, one, two or three times, typically three times before the less frequent administration dose(s) is (are) administered. In one embodiment of the invention, the first individual doses are administered at one month intervals (e.g., about 3 individual doses). The second dose is administered less frequently, e.g., at three month intervals (e.g., about 6 individual doses). In one aspect of the invention, the second individual doses are administered beginning three months after the number of first individual doses.

In one embodiment of the invention, the number of first individual doses and the number of second individual doses are administered over a time period of about 2 years. Shorter and longer time periods of 2 years are also included in the invention. In one aspect, the first individual dose is administered at month 0, 1 and 2. In another aspect, the second individual dose is administered at month 5, 8, 11, 14, 17, 20 and 23. In one example, the first individual dose is administered at month 0, 1, and 2 and the second individual dose is administered at month 5, 8, 11, 14, 17, 20 and 23.

Another aspect of the invention is the treatment of an intraocular neovascular disease, e.g., wet form AMD, by administering to a mammal, preferably a human patient, a number of first individual doses of a compound, e.g., a VEGF antagonist, followed by administering a number of second individual doses of the compound, where the number of second individual doses are administered less frequently than the number of first individual doses. This aspect of the invention is different than previous dosing methods for the treatment of such diseases which generally treat with regularly spaced, even doses of a therapeutic compound. For example, the Ranibizumab (rhuFab V2), which is an antihuman VEGF, affinity-matured Fab has been administered in equal monthly (about 28 days) doses of 0.3 mg or 0.5 mg. In contrast, the method of the invention provides a number of first individual doses which are typically evenly spaced follow by a number of second individual doses that are less frequently administered.

The patient receives an initial dose of the VEGF antagonist. Since the VEGF antagonist treatment effects are maintained for more than a month, the patient can receive less frequent doses of the therapeutic compound in subsequent doses. However, it is possible to give more frequent doses, within the scope of the invention, to patients who do not experience effects on first administration.

The dosage amount depends on the specific disease or condition which is treated and can be readily determined using known dosage adjustment techniques by a physician having ordinary skill in treatment of the disease or condition. The dosage amount will generally lie with an established therapeutic window for the therapeutic compound which will provide a therapeutic effect while minimizing additional morbidity and mortality. Typically, therapeutic compounds are administered in a dosage ranging from 0.001 mg to about 100 mg per dose, preferably 0.1-20 mg.

Also within the scope of the present invention are additional doses, which may be administered after the number of first individual doses and after the number of second individual doses. For example, an additional, third set of doses can be administered.

Typically, the therapeutic compound used in the methods of this invention is formulated by mixing it at ambient temperature at the appropriate pH, and at the desired degree of purity, with physiologically acceptable carriers, i.e., carriers that are non-toxic to recipients at the dosages and concentrations employed. The pH of the formulation depends mainly on the particular use and the concentration of antagonist, but preferably ranges anywhere from about 3 to about 8. Where the therapeutic compound is an anti-VEGF antibody (e.g., ranibizumab), a suitable embodiment is a formulation at about pH 5.5.

The therapeutic compound, e.g. an anti-VEGF antibody, for use herein is preferably sterile. Sterility can be readily accomplished by sterile filtration through (0.2 micron) membranes. Preferably, therapeutic peptides and proteins are stored as aqueous solutions, although lyophilized formulations for reconstitution are acceptable.

The therapeutic compound may be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the time scheduling of administration, and other factors known to medical practitioners. The "therapeutically effective amount" of the therapeutic compound to be administered is governed by such considerations, and is the minimum amount necessary to prevent, ameliorate, or treat an intraocular neovascular disease.

The therapeutic compound for treatment of an intraocular neovascular disease is typically administered by ocular, intraocular, and/or intravitreal injection. Other methods administration by also be used, which includes but is not limited to, topical, parenteral, subcutaneous, intraperitoneal, intrapulmonary, intranasal, and intralesional administration. Parenteral infusions include intramuscular, intravenous, intraarterial, intraperitoneal, or subcutaneous administration. As described herein, the therapeutic compound for treatment of an intraocular neovascular syndrome may be formulated, dosed, and administered in a fashion consistent with good medical practice.

The efficacy of the treatment of the invention can be measured by various endpoints commonly used in evaluating intraocular neovascular diseases. For example, vision loss can be assessed. Vision loss can be evaluated by, but not limited to, e.g., measuring by the mean change in best correction visual acuity (BCVA) from baseline to a desired time point (e.g., where the BCVA is based on Early Treatment Diabetic Retinopathy Study (ETDRS) visual acuity chart and assessment at a test distance of 4 meters), measuring the proportion of subjects who lose fewer than 15 letters in visual acuity at a desired time point compared to baseline, measuring the proportion of subjects who gain greater than or equal to 15 letters in visual acuity at a desired time point compared to baseline, measuring the proportion of subjects with a visual-acuity Snellen equivalent of 20/2000 or worse at a desired time point, measuring the NEI Visual Functioning Questionnaire, measuring the size of CNV and amount of leakage of CNV at a desired time point, e.g., by fluorescein angiography, etc. Ocular assessments can be done, e.g., which include, but are not limited to, e.g., performing eye exam, measuring intraocular pressure, assessing visual acuity, measuring slitlamp pressure, assessing intraocular inflammation, etc.

Any compound which binds to VEGF or a VEGF receptor and reduces the severity of symptoms or conditions associated with an intraocular neovascular disease may be used in this embodiment of the invention. Preferred compounds are peptide or protein compounds, more preferably are compounds which are or which contain an antibody or fragment thereof or which are fusions to an antibody fragment such as an immunoadhesin. Particularly preferred compounds are anti-VEGF antibodies or compounds containing fragments thereof.

VEGF is expressed in a variety of cells in the normal human retina. Co-localization of VEGF mRNA and protein is observed in the ganglion cell, inner nuclear and outer plexiform layers, the walls of the blood vessels, and photoreceptors (Gerhardinger et al., Am J Pathol 152:1453-62 (1998)). Retinal pigment epithelium, Muller cells, pericytes, vascular endothelium, and ganglion cells all produce VEGF (Miller et al., Diabetes Metab Rev 13:37-50 (1997); and, Kim et al. Invest Ophthalmol Vis Sci 40:2115-21 (1999)).

Studies have documented the immunohistochemical localization of VEGF in surgically resected CNV membranes from AMD patients. Kvanta et al. (1996) demonstrated the presence of VEGF mRNA and protein in RPE cells and fibroblast like cells. *See* Kvanta et al., Invest Ophthalmol Vis Sci 37:1929-34 (1996). Lopez et al. (1996) noted that the RPE cells that were strongly immunoreactive for VEGF were present primarily in the highly vascularized regions of CNV membranes, whereas the RPE cells found in fibrotic regions of CNV membranes showed little VEGF reactivity. *See* Lopez et al., Invest Ophthalmol Vis Sci 37:855-68 (1996). Kliffen et al. (1997) also demonstrated increased VEGF expression in RPE cells and choroidal blood vessels in maculae from patients with wet AMD compared with controls. *See* Kliffen et al., Br J Ophthalmol 81:154-62 (1997).

An increase in VEGF expression has been noted in experimental models of CNV in rats and in non human primates (Husain et al., Ophthalmology 104:124250 (1997); and, Yi et al. Vascular endothelial growth factor expression in choroidal neovascularization in rats. Graefes Arch Clin Exp Ophthalmol 235:313-9 (1997)). In addition, transgenic mice with increased VEGF expression in photoreceptors (Okamoto et al. 1997, *supra)* or retinal pigment epithelium (Schwesinger et al., AM J Pathol. 158(3):1161-72 (2001)) developed neovacularization reminiscent of CNV seen in humans with neovascular AMD. This further supports the involvement of VEGF in ocular neovascularization.

Of particular relevance to wet AMD are the angiogenic properties of VEGF, which have been demonstrated in a variety of in vivo models, including the chick chorioallantoic membrane (Leung et al., Science 246:1306-9 (1989); and, Plouet J, Schilling J, Gospodarowicz D. EMBO J 8:3801-6 (1989)), rabbit cornea (Phillips et al., In Vivo 8:961-5 (1994)), and rabbit bone (Connolly et al. J Clin Invest 84:1470―8 (1989a)). VEGF also functions as a survival factor for newly formed endothelial cells (Dvorak HF. N Engl J Med 315:1650―9 (1986); and, Connolly et al. J Biol Chem 264:20017―24 (1989b)). Consistent with pro survival activity, VEGF induces expression of the anti apoptotic proteins Bcl 2 and A1 in human endothelial cells (Connolly et al. J Biol Chem 264:20017―24 (1989b)).

VEGF has been shown to induce vascular leakage in guinea pig skin (Connolly et al. J Biol Chem 264:20017―24 ( 1989b)). Dvorak (1986) and colleagues (1987) proposed that an increase in microvascular permeability is a crucial step in angiogenesis associated with tumors and wound healing. Dvorak HF. N Engl J Med 315:1650-9 (1986); and, Dvorak et al., Lab Invest 57:673―86 (1987). A major function of VEGF in the angiogenic process can be the induction of plasma protein leakage. This effect would result in the formation of an extravascular fibrin gel, which serves as a substrate for endothelial cells. This activity can have relevance for AMD, as it is well established that permeability of the CNV membranes results in transudation of serum components beneath and into the retina, leading to serous macular detachment, macular edema and vision loss.

Thus, VEGF antagonists are good therapeutic compounds for treating intraocular neovascular diseases.

Many therapeutic compounds are well known to exert a therapeutic effect by binding to a selective cell surface marker or receptor or ligand. These known therapeutic compounds, e.g., anti-angiogenesis agents, are apparent to one having ordinary skill in the art and may be used in the method of the present invention. Suitable therapeutic compounds include non-peptidic organic compounds, preferably having a molecular weight less than about 1,000 g/mol, more preferably less than about 600 g/mol; peptide therapeutic compounds, generally containing 8 to about 200, preferably about 15 to about 150, more preferably about 20 to about 100 amino acid residues; and protein therapeutic compounds, generally having secondary, tertiary and possibly quaternary structure. Suitable peptides compounds can be prepared by known solid-phase synthesis or recombinant DNA technology which are well known in the art.

A particularly preferred method of selecting a peptide compound is through the use of phage display technology. Using known phage display methods, libraries of peptides or proteins are prepared in which one or more copies of individual peptides or proteins are displayed on the surface of a bacteriophage particle. DNA encoding the particular peptide or protein is within the phage particle. The surface-displayed peptides or proteins are available for interaction and binding to target molecules which are generally immobilized on a solid support such as a 96-well plate or chromatography column support material. Binding and/or interaction of the display peptide or protein with a target molecule under selected screening conditions allows one to select members of the library which bind or react with the target molecule under the selected conditions. For example, peptides which bind under particular pH or ionic conditions may be selected. Alternatively, a target cell population can be immobilized on a solid surface using known techniques and the peptide or protein phage library can be panned against the immobilized cells to select peptides or proteins which bind to cell surface receptors on the target cell population. Phage display techniques are disclosed, for example, in U.S. Pat. Nos. 5,750,373; 5,821,047; 5,780,279; 5,403,484; 5,223,407; 5,571,698; and others.

One category of polypeptide compounds, are compounds containing an antibody or a fragment thereof which immunologically recognize and bind to cell surface receptors or ligands. Methods of preparing antibodies are well known in the art and have been practiced for many years. Suitable antibodies may be prepared using conventional hybridoma technology or by recombinant DNA methods. Preferred antibodies are humanized forms of non-human antibodies. Alternatively, antibodies may be prepared from antibody phage libraries using methods described, for example, in U.S. Pat. Nos. 5,565,332; 5,837,242; 5,858,657; 5,871,907; 5,872,215; 5,733,743, and others. Suitable compounds include full-length antibodies as well as antibody fragments such as Fv, Fab, Fab' and F (ab')₂ fragments which can be prepared by reformatting the full length antibodies using known methods.

Additional preferred polypeptide therapeutic compounds are immunoadhesin molecules also known as hybrid immunoglobulins. These polypeptides are useful as cell adhesion molecules and ligands and also useful in therapeutic or diagnostic compositions and methods. An immunoadhesin typically contains an amino acid sequence of a ligand binding partner protein fused at its C-terminus to the N-terminus of an immunoglobulin constant region sequence. Immunoadhesins and methods of preparing the same are described in U.S. Pat. Nos. 5,428,130; 5,714,147; 4,428,130; 5,225,538; 5,116,964; 5,098,833; 5,336,603; 5,565,335; etc.

### Pharmaceutical Compositions

Therapeutic compounds of the invention used in accordance with the present invention are prepared for storage by mixing a polypeptide(s) having the desired degree of purity with optional pharmaceutically acceptable carriers, excipients or stabilizers *(*Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN™, PLURONICS™ or polyethylene glycol (PEG).

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations may be prepared. In one embodiment of the invention, an intraocular implant can be used for providing the VEGF antagonist. Suitable examples of sustained-release preparations include semipermeable matrices of solid hydrophobic polymers containing a polypeptide of the invention, which matrices are in the form of shaped articles, *e.g.* films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37 °C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### EXAMPLES

It is understood that the examples and embodiments described herein are for illustrative purposes only and that various modifications or changes in light thereof will be suggested to persons skilled in the art and are to be included within the spirit and purview of this application and scope of the appended claims.

### Example 1: Dosing Regiment

This study assesses the efficacy and safety of intravitreal injections of VEGF antagonist (e.g., ranibizumab) administered monthly for 3 doses followed by doses every 3 months compared with sham injections administered at the same schedule in subjects with primary or recurrent subfoveal choroidal neovascularization (CNV) with or without a classic CNV component secondary to AMD.

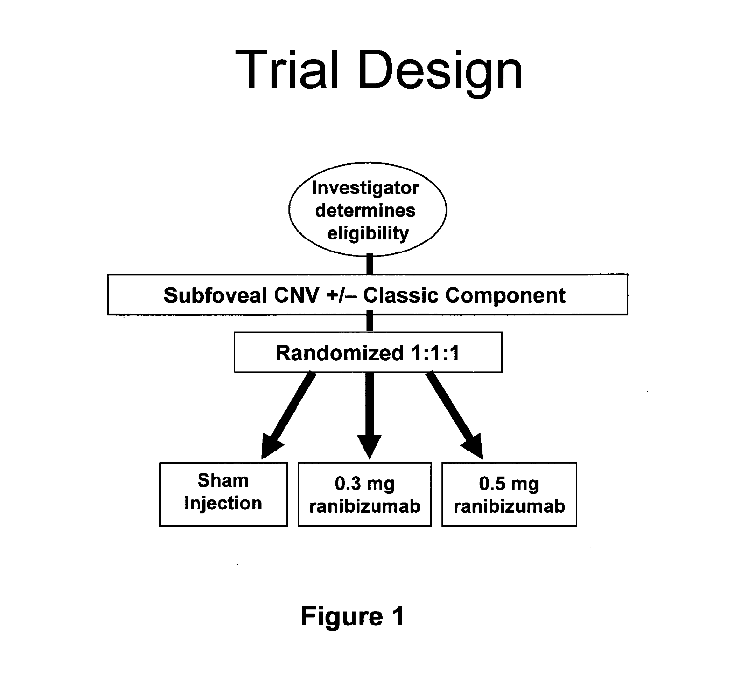

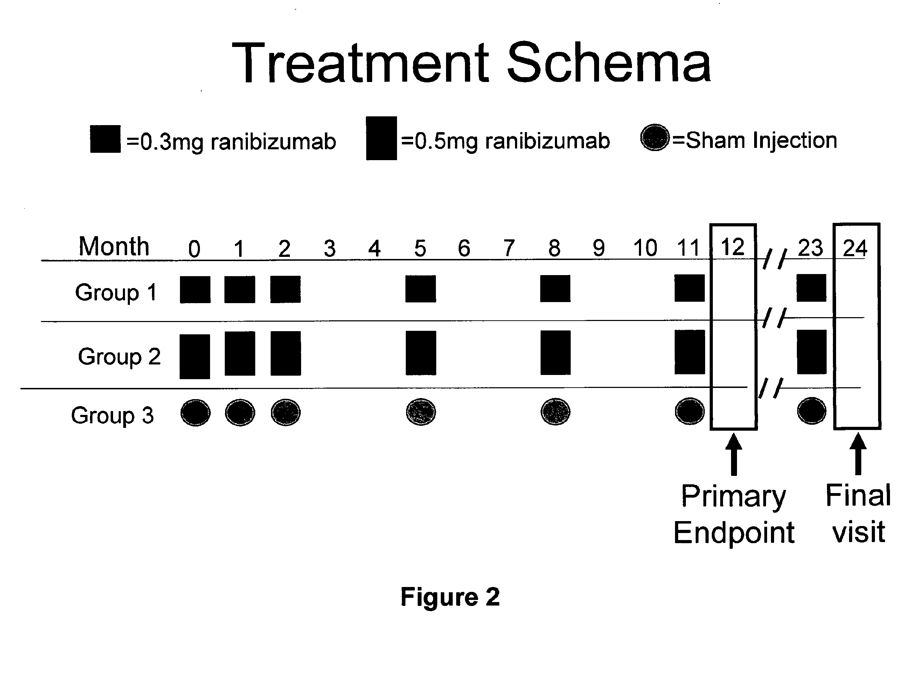

In this study, two treatment groups receive multiple intravitreal doses of VEGF antagonist from 0.3 mg to 0.5 mg for 24 months. See **Figure 1**. Each dose of VEGF antagonist is administered every month for 3 doses (Day 0, Month 1 and Month 2) followed by doses every 3 months (Months 5, 8, 11, 14, 17, 20, and 23) until study termination. See **Figure 2****.** Subjects randomized to sham injections follow the same schedule as subjects receiving ranibizumab. During the 24 month study period, a total of 10 ranibizumab or 10 sham injections can be administered. Typically, the dosing does not occur earlier than 14 days after the previous treatment. If a dose is withheld or is missed, it may be optionally administered within 14 days following the previous treatment during the monthly injection period or within 45 days after the previous treatment during the 3-month dosing period. A maximum of 10 doses of study drug is administered during this study. Ranibizumab is administered in one eye only (study eye) during this study.

An example of a VEGF antagonist is ranibizumab (LUCENTIS™). Ranibizumab (rhuFab V2) is a humanized, affinity-matured anti-human VEGF Fab fragment. Ranibizumab is produced by standard recombinant technology methods in Escherichia coli expression vector and bacterial fermentation. Ranibizumab is not glycosylated and has a molecular mass of -48,000 daltons. See WO98/45331 and US20030190317.

*Ranibizumab Injection:* For intravitreal administration, the study drug, ranibizumab, is supplied in a liquid-filled vial of ranibizumab. Each vial contains 0.7 mL of either 6 mg/mL (0.3 mg dose level) or 10 mg/mL (0.5-mg dose level) of ranibizumab aqueous solution (pH 5.5) with 10 mM of histidine, 100 mg/mL of trehalose, and 0.01% polysorbate 20. All study drug is stored at 2°C―8°C (36°F―46°F), and should not be frozen. Drug should be protected vials from direct sunlight.

Procedures are implemented to minimize the risk of potential adverse events associated with serial intraocular injections (e.g., endophthalmitis). Aseptic technique is observed for the injection tray assembly, anesthetic preparation and administration, and study drug preparation and administration.

Intravitreal injections are performed by the injecting physician(s) following the slitlamp examination. After thorough cleansings of the lid, lashes, and periorbital area with an antiseptic, local anesthesia and antimicrobials can administered prior to study drug injection.

A 30 gauge, ½-inch needle attached to a low volume (e.g., tuberculin) syringe containing 50 µL of study drug solution is inserted through the pre anesthetized conjunctiva and sclera, approximately 3.5―4.0 mm posterior to the limbus, avoiding the horizontal meridian and aiming toward the center of the globe. The injection volume should be delivered slowly. The needle is then be removed slowly to ensure that all drug solution is in the eye. Immediately following the intraocular injection, antimicrobial drops can be administered and the subject is instructed to self-administer antimicrobial drops four times daily for 3 days following each intraocular injection of ranibizumab. The scleral site for subsequent intravitreal injections should be rotated.

*Sham Injection:* The injecting physician(s) performs the same pre-injection cleansing and anesthetizing procedures (including subconjunctival injection of anesthesia) outlined above for subjects receiving ranibizumab. An empty syringe without a needle is used in the sham injection. The injecting physician(s) mimics an intraocular injection by making contact with the conjunctiva and applying pressure without the needle. Immediately following the sham injection, the injecting physician(s) performs the same post-injection procedures as those performed on subjects receiving ranibizumab.

*Pre-Injection Procedures for All Subjects (Raninizumab or Sham Injection):* The following procedures can be implemented to minimize the risk of potential adverse events associated with serial intravitreal injections (e.g., endophthalmitis). Aseptic technique is observed for injection tray assembly, anesthetic preparation, and study drug preparation and administration. The following procedures (except where noted) can be conducted by the physician performing the intravitreal injection of ranibizumab or sham injection. Subjects receive antimicrobials (e.g., ofloxacin ophthalmic solution or trimethoprim polymyxin B ophthalmic solution) for self-administration four times daily for 3 days prior to treatment.
- The supplies are assembled and and a sterile field is prepared. Supplies can include 10% povidone iodine swabs, sterile surgical gloves, 4X4 sterile pads, pack of sterile cotton tipped applicators, eyelid speculum, sterile ophthalmic drape, 0.5% proparacaine hydrochloride, 5% povidone iodine ophthalmic solution, 1% lidocaine for injection, ophthalmic antimicrobial solution (e.g., ofloxacin ophthalmic solution or trimethoprim polymyxin B ophthalmic solution, single-use vial), and injection supplies.
- 2 drops of 0.5% proparacaine hydrochloride are instilled into the study eye, followed by 2 drops of a broad spectrum antimicrobial solution (e.g., ofloxacin ophthalmic solution or trimethoprim polymyxin B ophthalmic solution, single-use vial).
- The periocular skin and eyelid of the study eye are disinfected in preparation for injection. The eyelid, lashes, and periorbital skin are scrubbed with 10% povidone iodine swabs, starting with the eyelid and lashes and continuing with the surrounding periocular skin. The eyelid margins and lashes are swabbed, e.g., in a systematic fashion, from medial to temporal aspects.
- A sterile ophthalmic drape can be placed to isolate the field, and the speculum can be placed underneath the eyelid of the study eye.
- 2 drops of 5% povidone iodine ophthalmic solution are instilled in the study eye, making sure the drops cover the planned injection site on the conjunctiva.
- Wait 90 seconds.
- A sterile cotton-tipped applicator is saturated with 0.5% proparacaine hydrochloride drops and the swab is held against the planned intravitreal injection site for 10 seconds in preparation for the subconjunctival injection of 1% lidocaine hydrochloride ophthalmic solution for injection (without epinephrine).
- 1% lidocaine (without epinephrine) is injected subconjunctivally.
- A sterile 4X4 pad in a single wipe can be used to absorb excess liquid and to dry the periocular skin.
- The subject is instructed to direct gaze away from syringe prior to ranibizumab or sham injection.

*Ranibizumab Preparation and Administration Instructions:* The ranibizumab injection can be prepared as herein. Dose solutions are typically prepared immediately before dosing. Dose solutions are typically for single use only.

After preparing the study eye as outlined above, 0.2 mL ranibizumab dose solution is withdrawn through a 5-µm filter needle. The filter needle is removed and replaced with a 30-gauge, ½ inch Precision Glide® needle, and excess ranibizumab is expelled so that the syringe contains 0.05 mL ranibizumab solution. The syringe is inserted through an area 3.5―4.0 mm posterior to the limbus, avoiding the horizontal meridian and aiming toward the center of the globe. The injection volume should be delivered slowly. The needle is then removed slowly to ensure about all drug solution is in the eye. The scleral site for subsequent intravitreal injections should be rotated. Refer to next section for detailed post injection procedures.

The subject can be monitored with a finger count test for the study eye within, e.g., 15 minutes of the ranibizumab injection. A measurement of intraocular pressure of the study eye can be obtained, e.g., 60 minutes (±10 minutes) following the ranibizumab injection.

*Post-Injection Procedures for All Subjects:* Immediately following the ranibizumab or sham injection, 2 drops of antimicrobial drops (e.g., ofloxacin ophthalmic solution or trimethoprim polymyxin B ophthalmic solution, single-use vial) are instilled in the study eye. The subject is instructed to self-administer antimicrobial drops (e.g., ofloxacin ophthalmic solution or trimethoprim polymyxin B ophthalmic solution, single-use vial) four times daily for 3 days following each injection (ranibizumab or sham).

*Preparation and Administration of the Sham Injection:* See above for detailed instructions for pre-injection procedures.

Subjects receiving sham injections do not receive an actual injection of study drug. The physician follows the procedures for cleansing and anesthetizing the study eye as outlined above. The subject should be instructed to direct his or her gaze away from the syringe prior to administration of the sham injection. The tuberculin syringe plunger is withdrawn to the 0.05 mL mark on the syringe, the hub of the syringe―without the needle—is then placed against the pre-anesthetized conjunctival surface. The syringe hub is pressed firmly against the globe and then the plunger is slowly depressed, mimicking the action of an intravitreal injection.

For subsequent sham injections, the procedure of rotating the location of the injection site, as is done with ranibizumab injections is followed. See above for detailed post-injection procedures.

The subject can be monitored using a finger count test within, e.g., 15 minutes of the sham injection. A measurement of intraocular pressure can be obtained, e.g., 60 minutes (±10 minutes) following the sham injection.

Safety is assessed by the incidence of ocular and non-ocular adverse events, including but not limited to, serious adverse events, ocular assessments, deaths, laboratory test results, vital signs, antibodies to Raninizumab, intraocular inflammation, visual acuity, intraocular pressure, slitlamp pressure, indirect ophthalmoscopy, fluorescein angiography, fundus photography, vitreous hemorrhage, sensory rhegmatogenous retinal break or detachment (including macular hole), subfoveal hemorrhage, local or systemic infection, intraocular surgery, etc. In one embodiment, if verteporfin PDT was given within the last 28 days, the ranibizumab/sham injection is withheld. Efficacy is assessed by changes in preventing vision loss, e.g., measured by the mean change in best correction visual acuity (BCVA) from baseline to 12 months or 24 months (where the BCVA is based on the Early Treatment Diabetic Retinopathy Study (ETDRS) visual acuity chart and assessment at a test distance of 4 meters), other means include but are not limited to measuring the proportion of subjects who lose fewer than 15 letters in visual acuity at 12 months or 24 months compared to baseline, measuring the proportion of subjects who gain greater than or equal to 15 letters in visual acuity at 12 months or 24 months compared to baseline, measuring the proportion of subjects with a visual-acuity Snellen equivalent of 20/2000 or worse at 12 months or 24 months, measuring the NEI Visual Functioning Questionnaire, measuring the size of CNV and amount of leakage of CNV at 12 months or 24 months, e.g., by fluorescein angiography.

The specification is considered to be sufficient to enable one skilled in the art to practice the invention. Various modifications of the invention in addition to those shown and described herein will become apparent to those skilled in the art from the foregoing description and fall within the scope of the appended claims. All publications, patents, and patent applications cited herein are hereby incorporated by reference in their entirety for all purposes.

The following numbered clauses, which form part of the description, not the claims, define further aspects and embodiments of the invention.
1. A method for treating wet form age-related macular degeneration in a mammal, comprising the steps of:
   a) administering to the mammal a number of first individual doses of an VEGF antagonist; and
   b) administering to the mammal a number of second individual doses of the VEGF antagonist, wherein the second individual doses are administered less frequently than the first individual doses.
2. The method of para 1, wherein the mammal is a human.
3. The method of para 1, wherein the administration is ocular.
4. The method of para 3, wherein the administration is intraocular.
5. The method of para 4, wherein the administration is intravitreal.
6. The method of para 1, wherein the VEGF antagonist is an anti-VEGF antibody.
7. The method of para 6, wherein the anti-VEGF antibody is a full length anti-VEGF antibody.
8. The method of para 6, wherein the anti-VEGF antibody is an antibody fragment.
9. The method of para 6, wherein the anti-VEGF antibody is a Fab antibody fragment.
10. The method of para 8, wherein the antibody fragment is Y0317.
11. The method of para 1, wherein the first individual doses are administered at one month intervals.
12. The method of para 1, wherein the second individual doses are administered at three month intervals.
13. The method of para 1, wherein the second individual doses are administered beginning three months after the number of first individual doses.
14. The method of para 1, wherein the number of second individual doses are administered to the mammal during a period of at least 22 months following the number of first individual doses.
15. The method of para 1, wherein the number of the first individual doses comprises about 3 individual doses.
16. The method of para 1, wherein the number of the second individual doses comprises about 6 individual doses.
17. The method of para 1, wherein the number of first individual doses and the number of second individual doses are administered over a time period of about 2 years.
18. The method of para 1, wherein the first individual dose is administered at month 0, 1 and 2.
19. The method of para 1, wherein the second individual dose is administered at month 5, 8, 11, 14, 17, 20 and 23.
20. The method of para 1, wherein the first individual dose is administered at month 0, 1, and 2 and the second individual dose is administered at month 5, 8, 11, 14, 17, 20 and 23.
21. A method for treating intraocular neovascular disease, comprising:
   administering to a mammal a number of first individual doses of an VEGF antagonist; followed by,
   administering to the mammal a number of second individual doses of the antagonist, wherein the second individual doses are administered less frequently than the first individual doses.

## Claims

1. A VEGF antagonist for use in a method for treating diabetic macular edema, the method comprising:
a) administering to a mammal a number of first individual doses of the VEGF antagonist; followed by
b) administering to the mammal a number of second individual doses of the antagonist,
wherein the second individual doses are administered less frequently than the first individual doses.

2. The VEGF antagonist of claim 1, wherein the mammal is a human.

3. The VEGF antagonist of claim 1, wherein the administration is ocular.

4. The VEGF antagonist of claim 3, wherein the administration is intraocular.

5. The VEGF antagonist of claim 4, wherein the administration is intravitreal.

6. The VEGF antagonist of claim 1, wherein the VEGF antagonist is an anti-VEGF antibody.

7. The VEGF antagonist of claim 6, wherein the anti-VEGF antibody is a full length anti-VEGF antibody.

8. The VEGF antagonist of claim 6, wherein the anti-VEGF antibody is an antibody fragment.

9. The VEGF antagonist of claim 6, wherein the anti-VEGF antibody is a Fab antibody fragment.

10. The VEGF antagonist of claim 8, wherein the antibody fragment is Y0317.

11. The VEGF antagonist of claim 1, wherein the first individual doses are administered at one month intervals.

12. The VEGF antagonist of claim 1, wherein the second individual doses are administered at three month intervals.

13. The VEGF antagonist of claim 1, wherein the second individual doses are administered beginning three months after the number of first individual doses.

14. The VEGF antagonist of claim 1, wherein the number of second individual doses are administered to the mammal during a period of at least 22 months following the number of first individual doses.

15. The VEGF antagonist of claim 1, wherein the number of the first individual doses comprises about 3 individual doses.

16. The VEGF antagonist of claim 1, wherein the number of the second individual doses comprises about 6 individual doses.

17. The VEGF antagonist of claim 1, wherein the number of first individual doses and the number of second individual doses are administered over a time period of about 2 years.

18. The VEGF antagonist of claim 1, wherein the first individual dose is administered at month 0, 1 and 2.

19. The VEGF antagonist of claim 1, wherein the second individual dose is administered at month 5, 8, 11, 14, 17, 20 and 23.

20. The VEGF antagonist of claim 1, wherein the first individual dose is administered at month 0, 1, and 2 and the second individual dose is administered at month 5, 8, 11, 14, 17, 20 and 23.

21. Use of a VEGF antagonist in the manufacture of a medicament for use in a method of treating diabetic macular edema, the method comprising:
a) administering to a mammal a number of first individual doses of the VEGF antagonist; followed by
b) administering to the mammal a number of second individual doses of the antagonist,
wherein the second individual doses are administered less frequently than the first individual doses.

22. Use according to claim 21, wherein the mammal, administration and antagonist are as defined in any one of claims 2 to 20.

23. A sustained-release intraocular implant comprising a semipermeable matrix of solid hydrophobic polymer containing a VEGF antagonist polypeptide, wherein the matrix is in the form of a film.
